# EUROPEAN PATENT SPECIFICATION

(11) **EP 4 008 292 B1**
(45) Date of publication and mention of the grant of the patent: **25.11.2026**
(21) Application number: 21212405.1
(22) Date of filing: 03.12.2021
(51) Int. Cl.: A61F 2/28, A61F 2/30

(54) **NESTED HARD TISSUE REPLACEMENT IMPLANTS**
VERSCHACHTELTE ERSATZIMPLANTATE AUS HARTGEWEBE
IMPLANTS DE REMPLACEMENT DE TISSUS DURS IMBRIQUÉS

(30) Priority: 03.12.2020 US 202063120869 P
(43) Date of publication of application: 08.06.2022
(73) Proprietor: Zimmer Biomet CMF And Thoracic, LLC, Jacksonville, Florida 33218 (US)
(72) Inventor: BILLARD, Max Holland, Jacksonville, 32207 (US); FRANK, Zachary, Jacksonville, 32256 (US); ZBOROWSKI, Laura, Jacksonville, 32246 (US); HAUSMAN, Adam Webster, Jacksonville, 32218 (US)
(74) Representative: Allen, Caroline Margaret

(56) References cited:
- WO-A1-2016/024248
- WO-A1-2018/076075
- US-A1- 2017 027 629
- US-A1- 2019 209 328

## Description

### BACKGROUND

Hard tissue replacement implants for areas such as bone are often required to replace sections of bone damaged by tumors or infection. For example, hard tissue replacements can be used to replace sections of cranium or skull when tumorous portions of the cranium are removed. The sections can be replaced by implants such as plates that can be secured to the remaining healthy skull to cover the void left by removing compromised bone.

US 2019/209328 A1 discloses a universal low-profile intercranial assembly which includes a mounting plate and a low profile intercranial device composed of a static cranial implant and an interdigitating functional neurosurgical implant.

A hard tissue implant assembly according to the invention is defined in claim 1.

### BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.
FIG. 1A illustrates a side perspective view of a skull.
FIG. 1B illustrates a side isometric view of a skull.
FIG. 2A illustrates a top view of a hard tissue replacement implant.
FIG. 2B illustrates a top view of a hard tissue replacement implant.
FIG. 2C illustrates a cross-sectional view of an implant and a cranium.
FIG. 3A illustrates a top view of a hard tissue replacement implant.
FIG. 3B illustrates a top view of a hard tissue replacement implant.
FIG. 4 illustrates a top view of a hard tissue replacement implant.
FIG. 5 illustrates a top view of a hard tissue replacement implant.
FIG. 6 illustrates a flow schematic for installing a hard tissue implant.

### DETAILED DESCRIPTION

Hard tissue replacement implants for areas such as bone are often required to replace sections of bone damaged by tumors or infections. During one type of procedure, a damage section is removed to an estimated first margin and one or more samples are taken to determine if the first margin is free of cancerous cells. If it is, an implant can be secured to the cranium (or bone) to replace the resected bone. If the margin is not free of cancerous cells, the surgeon typically goes back to resect more bone to a second margin and samples are tested again. The process can be repeated to ensure the margins are all free of cancerous cells. Because this process can be time consuming and can be costly due to acquiring a set of implants of various sizes, surgeons often make a conservative operative plan to ensure that the margins will be free of cancerous cells after the first resection and one implant size can be ordered. However, while this plan can save time and cost, such a conservative approach can result in more healthy bone being taken than is required.

The inventors have recognized that this procedure can be modified to potentially save more healthy bone by using a nesting hard tissue replacement implant assembly. The first (inner) implant can be selected for replacement of a resected bone at a first margin according to a pre-operative plan where the first implant is sized to replace the first resected portion at the first margin, where the first margin is likely to be cancer-free but where the first margin is less conservative than one of the conservative approach of the strategy discussed above. Following resection, edge samples can be analyzed to ensure that the edges are free of cancerous cells.

When analysis confirms that the margins are safe and free of cancerous cells, the inner implant can be installed to replace the resected portion. However, when the analysis determines that the margins are not free of cancerous cells, the surgeon can resect the bone a second time to create a second margin that is larger than the first margin, such as according to a pre-operative plan where the second margin is selected to be more conservative and very likely to be free of cancerous cells. The outer implant can then be secured to the cranium (bone) and the inner implant can be nested within the outer implant and secured to the outer implant before completing a normal closing procedure. Such a process can help to save as much healthy bone as the surgeon thinks is possible while helping to minimize iteration of resection and testing and while using a single implant system to help reduce costs.

Though the invention is discussed with respect to cancerous bone replacement. The same methods can be applied for infected bone requiring replacement.

The above discussion is intended to provide an overview of subject matter of the present patent application. It is not intended to provide an exclusive or exhaustive explanation of the invention. The description below is included to provide further information about the present patent application.

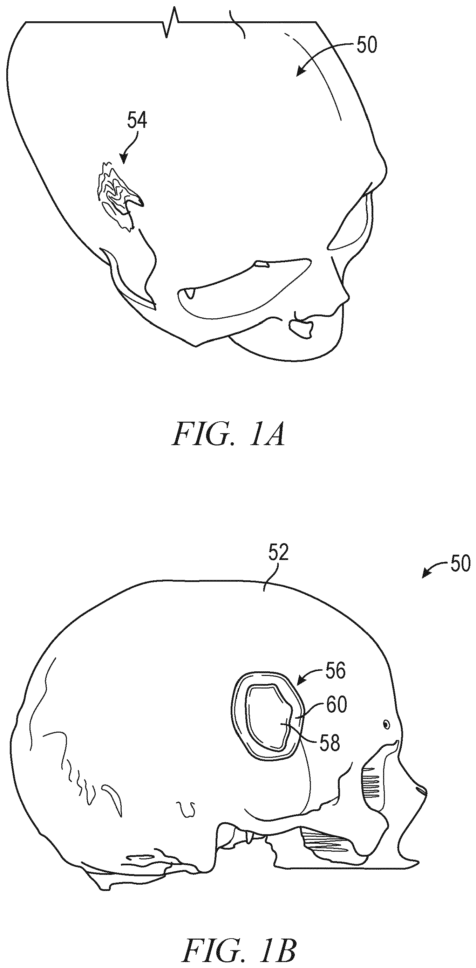

FIG. 1A illustrates a side perspective view of a skull 50 including a cranium bone portion 52 and a tumorous portion 54 of the cranium 52. FIG. 1B illustrates a side isometric view of the skull 50 showing margin plan 56 for the procedure. The margin plan 56 can include an inner margin portion 58 that is likely to be free of cancerous cells and is sized to receive an inner implant. The margin plan 56 can include an outer margin portion 60 that is larger than the inner margin 58 and is sized to receive an outer implant. Which implant is used can be based on edge sample analysis, as discussed in further detail below.

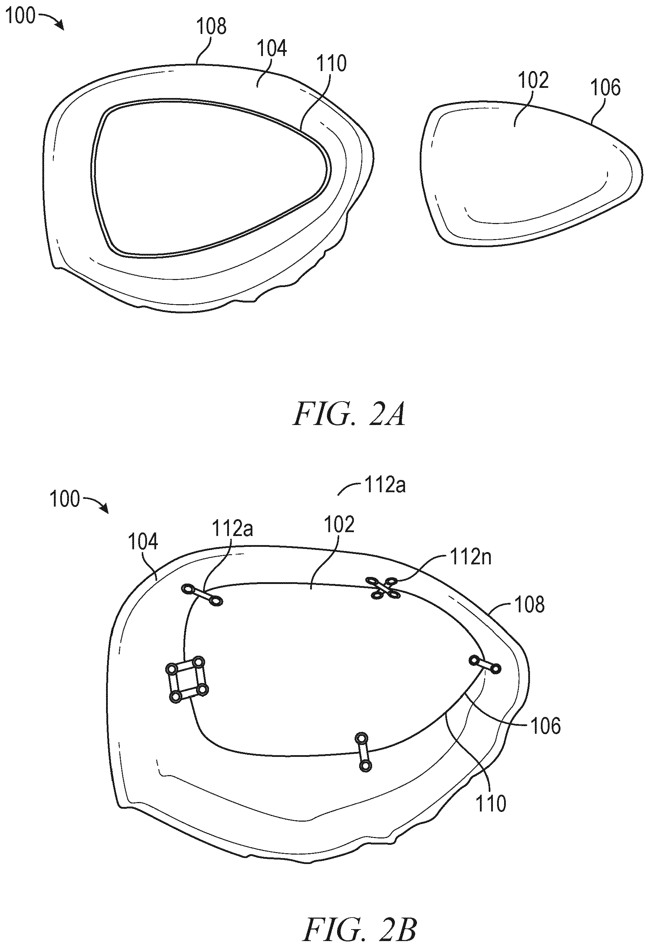

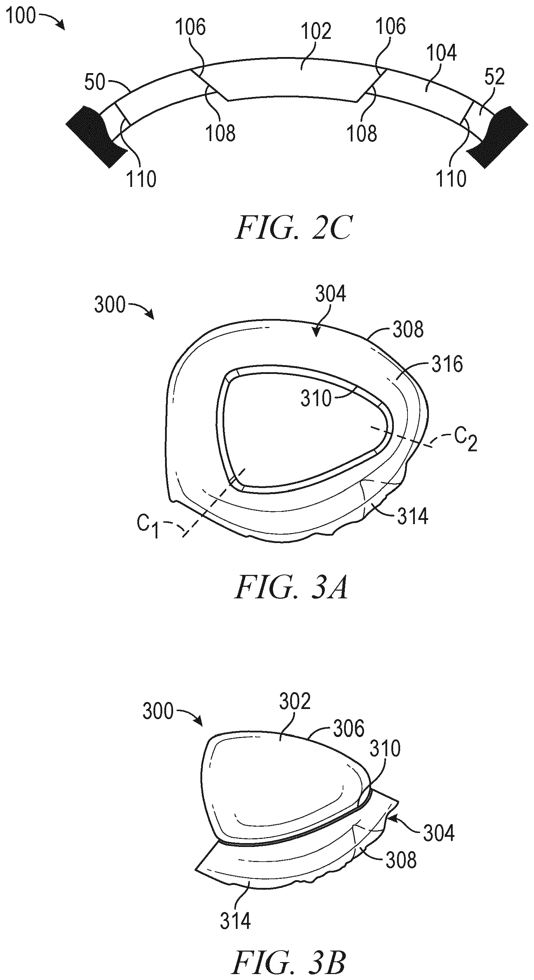

FIG. 2A illustrates a top view of a hard tissue replacement implant assembly 100 (referred to as implant assembly 100). FIG. 2B illustrates a top view of the hard tissue replacement implant assembly 100. FIG. 2C illustrates a cross-sectional view of the implant assembly 100 and a cranium 52. FIGS. 2A-2C are discussed together below.

The implant assembly 100 includes an inner (or first) implant 102 and an outer (or second) implant 104. The inner implant defines an outer edge 106. The outer implant 104 defines an outer edge 108 and an inner edge 110. The inner implant 102 and the outer implant 104 can be made of biocompatible materials such as one or more of stainless steels, cobalt-chromium, titanium variations, polyether ketone ketone (PEKK), polyether ether ketone (PEEK), or the like.

The implant assembly 100 can be designed in a pre-operative planning procedure where a surgeon or physician selects a first margin region (such as the region 58 of FIG. 1B), that is likely to be free of cancerous cells. The physician can then select an outer margin region, such as the margin portion 60 of FIG. 1B, that is larger than the inner margin 58 and very likely to be free of cancerous cells. The inner implant 102 can be sized to replace hard tissue of the inner region 58 and the outer implant 104 can be sized such that the inner implant 102 can nest in the outer implant 104 and such that the inner implant 102 and outer implant 104 can be sized to, together, replace all of the hard tissue removed to create the outer margin 60. Such an implant assembly can help to preserve as much healthy bone as the surgeon thinks is possible while helping to minimize iteration of resection and testing and while using a single implant system to help reduce costs.

As shown in FIGS. 2A and 2C, the inner edge 110 of the outer implant 104 is beveled. And as shown in FIG. 2C, the outer edge 106 of the inner implant is beveled (or chamfered) complimentary to the bevel of the inner edge 110 of the outer implant 104. The bevels of the outer edge 106 and the inner edge 110 configures the inner implant 102 to nest within the outer implant 104, where outer surfaces of the inner implant 102 and the outer implant 104 are aligned or even where the inner implant 102 and the outer implant 104 meet, as shown in FIGS. 2B and 2C. The bevels of the outer edge 106 and the inner edge 110 are also configured (angled down and radially inwardly) to limit movement of the inner implant 102 through the inner implant 104 when the implants are in the nested orientation of FIG. 2B, which can help limit relatively movement of the implants and can help to enable simpler fastening of the implants.

As shown in FIG. 2B, the implants 102 and 104 can be secured together using fasteners 112a-112n, where the fasteners 112 can be secured to both the inner implant 102 and the outer implant 104. The fasteners 112 can also be used to secure the outer implant 104 to bone (such as the cranium 52). The fasteners 112 can be bone fasteners of any type (e.g., screws, plates, or pins).

FIG. 3A illustrates a top view of a hard tissue replacement implant assembly 300. FIG. 3B illustrates a top view of the hard tissue replacement implant assembly 300 (implant assembly 300). FIGS. 3A and 3B are discussed together below.

The implant assembly 300 can include an inner (or first) implant 302 and an outer (or second) implant 304. The inner implant can define an outer edge 306. The outer implant 304 can define an outer edge 308 and an inner edge 310. The outer implant 304 can also include a trimmed portion 314 and a sacrificial portion 316. Also shown in FIG. 3A are cut lines C1 and C2.

The implant assembly 300 of FIGS. 3A and 3B can be similar to the implant assembly 100 discussed above with respect to FIGS. 2A-2C; FIGS. 3A and 3B show another way that such implant assemblies can be used. For example, after the first margin resection is performed it may be determined that only a portion of the second margin must be removed. In such a case, the outer implant 304 can be trimmed to match the smaller resected second margin, such as by trimming the outer implant at cut lines C1 and C2. Cut lines C1 and C2 can be straight, which can help to simplify the implant trimming process and the bone resection process.

Then, the trimmed portion 314 of the outer implant 304 can be secured to bone to fill the void created by the second margin portion. The inner implant 302 can then be secured to the trimmed portion 314 and the cranium to fill the remainder of the void created by creation of the first margin portion. The sacrificial portion 316 can be discarded. Such a process can allow for a single implant assembly to be used to help fill voids of a variety of margin shapes and locations helping to conserve as much healthy bone as possible.

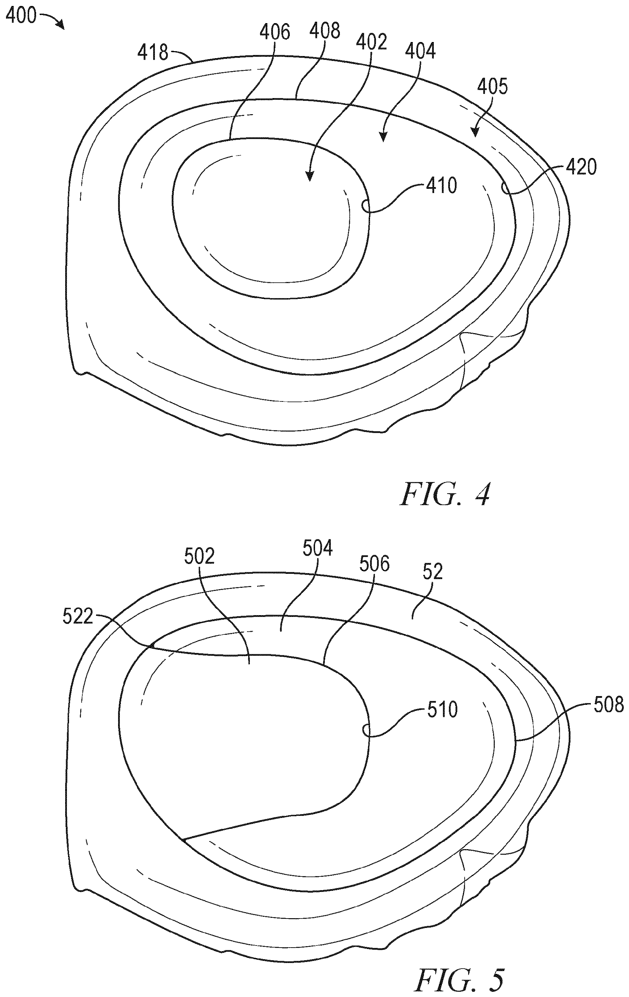

FIG. 4 illustrates a top view of a hard tissue replacement implant assembly 400. The implant 400 can include an inner (or first) implant 402 a middle (or second) implant 404, and an outer (or third) implant 405. The inner implant 402 can define an outer edge 406. The middle implant 404 can define an outer edge 408 and an inner edge 410. The outer implant 405 can define an outer edge 418 and an inner edge 420.

The implant assembly 400 can be similar to the implant assemblies 100 and 300 discussed above; the implant assembly 400 can include a third implant (the outer implant 405. Such an implant assembly can allow for a third margin region to be planned for in a case where a surgeon is trying to conserve as much bone as possible or where there is a lot of uncertainty as to where safe margins will be (for example due to a rapidly expanding cancer or infection). For example, the inner implant 402 can be used when the first margin region is free of cancerous cells, the inner implant 402 and at least a portion of the middle implant 404 can be used when the first margin region is not free of cancerous cells, and the inner implant 402 and at least a portion of the middle implant 404 and the outer implant 405 can be used when the second margin region is not free of cancerous cells.

The inner implant 402 can be configured to nest within the middle implant 404 and the middle implant 404 can be configured to nest within the outer implant 405 such that the inner implant 402 and the middle implant 404 nest within the outer implant 405. The edges (406, 408, 410, and 420) of the implants can all be beveled or chamfered (similar to the implant assembly 100) to allow for nesting. Such a nesting implant assembly can allow the implants to act as a single implant when secured to each other. FIG. 4 also shows that the implants 402 and 404 can have irregular and different shapes.

Also, in some examples, the implant assembly 400 can be manufactured to a standard size. For example, a three-piece assembly 400 can be manufactured to match contours of an average person's cranium for a portion (such as a lateral portion) using, for example, a dataset of dimensions for a large number of samples. The samples can be averaged and the inner implant 402, middle implant 404, and the outer implant 405 can each be manufactured based on the data. Such an implant assembly can be kept in stock for emergency procedures or can be used where cost reduction is more important than bone conservation. In some examples, average tumor or infection size and growth rate can be used to create the inner implant 402, middle implant 404, and the outer implant 405.

FIG. 5 illustrates a top view of a hard tissue replacement implant assembly 500. The implant assembly 500 can include an inner (or first) implant 502 and an outer (or second) implant 304. The inner implant can define an outer edge 506. The outer implant 504 can define an outer edge 508 and an inner edge 510.

The implant assembly 500 of FIG. 5 can be similar to the implant assemblies 100 and 300 discussed above; the outer implant 504 can form a U-shape. More specifically, the inner edge 510 of the outer implant can meet the outer edge 508 such that the outer implant 504 can include an opening 522 on one side such that the outer implant 504 forms a C-shape or a U-shape. The exact shape can vary such that the outer implant 504 is not a precise C-shape or U-shape but is not a complete shape such that it is open to one side allowing the outer edge 506 of the inner implant 502 to form a portion of an outer periphery of the implant assembly 500. Such an assembly can be useful where margins are less certain on one or more portions of the cranium 52.

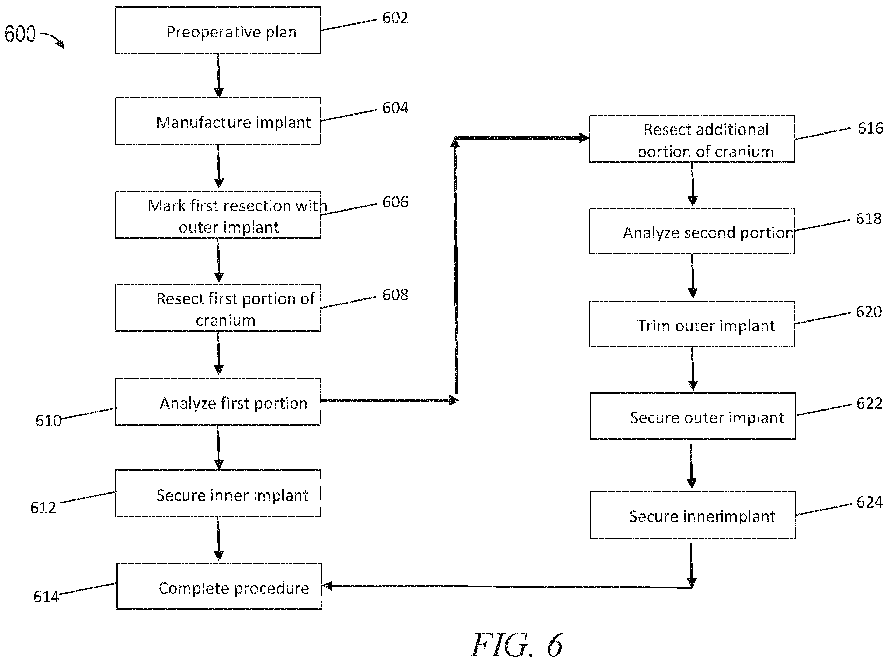

FIG. 6 illustrates a schematic view of the method 600, in accordance with at least one example of this disclosure. The method 600 can be a method of securing an implant assembly to bone. More specific examples of the method 600 are discussed below. The steps or operations of the method 600 are illustrated in a particular order for convenience and clarity; many of the discussed operations can be performed in a different sequence or in parallel without materially impacting other operations. The method 600 as discussed includes operations performed by multiple different actors, devices, and/or systems. It is understood that subsets of the operations discussed in the method 600 can be attributable to a single actor, device, or system could be considered a separate standalone process or method.

At step 602 a preoperative plan can be created, such as by a physician or surgeon where the inner implant (such as the implant 102) can be selected for replacement of a resection at a first margin where the first implant size is selected at a first margin that is likely to be cancer-free but with margins less conservative than in a second margin region. The outer implant (such as the implant 104) can be sized to replace bone of the second margin region. Similarly, a third, further, etc., implant can be sized for margins during the preoperative planning step.

At step 604 the implant can be manufactured based on the preoperative plan and the patient's anatomy such as by using a three-dimensional (3D) printing procedure (e.g., selective laser sintering). Such a manufacturing procedure can help reduce the likelihood of warping or mushrooming that can occur in the fabrication of a single large implant. The use of 3D printing also helps to reduce material waste over other machining alternatives.

The surgical procedure can begin at step 606 after the patient is prepped and an opening is created. At step 606 an inner edge of the outer implant (such as the edge 110) can be used to mark the first resection location on the cranium. Marking the resection in this manner can be beneficial because deformed portions (such as a protruding tumor) of the cranium can extend through the opening of the outer implant (e.g., opening of the outer implant 104), which can help to allow the resection lines for the first margin to be marked more accurately than by use of a flat template. At step 608 the first portion of the cranium can be resected to the first margin portion and at step 610 edge samples can be analyzed to determine whether they are free of cancerous cells.

When the samples are determined to be free of cancerous cells (and the first margin portion is therefore good or clear), step 612 can be performed where the inner implant (e.g., the inner implant 102) can be secured to the cranium and the procedure can be completed. When the edge samples are not free of cancerous cells, a second portion of the cranium can be resected at step 616. A portion of the second margin area can be resected or all of the area can be resected based on the results of the analysis from step 610. Then, samples (such as edge samples) from the second resection can be analyzed to determine if they are free of cancerous cells. If so, the outer implant (e.g., 304) can be trimmed at the step 620 based on the results and the trimmed portion (e.g., trimmed portion 314) can be secured to the cranium at step 622. Optionally, such as when multiple edge samples include tumorous cells, the entire second margin region can be removed and trimming of the outer implant can be skipped and the entire outer implant (e.g., 304) can be secured to the cranium at step 622. When the analysis of step 618 determines that the additional portion of the resection is not free of cancerous cells, the steps 616 and 618 can be repeated until margins free of cancerous cells is achieved.

Once the outer implant(s) are secured to the cranium, the inner implant(s) (e.g., 102, 302, or 402) can be nested within the outer implant and secured thereto. When a middle implant or implants is used, the middle implant(s) can be secured to the outer implant prior to securing the inner implant. Any of the implants can be secured using a plurality of fasteners. Then, at step 614, following either of steps 612 or 624, the procedure can be completed (including closing, etc.).

Such a procedure can help to save as much healthy bone as the surgeon thinks is possible while helping to minimize iteration of resection and testing and while using a single implant system to help reduce costs. Also, by using a smaller implant as the inner implant, surgeons are provided with a smaller access area for reentry, if required. Though the devices and procedures are discussed primarily with respect to replacement of cancerous cranium segments, they can be applied to replacement of infected bone or for replacement of fractured or deformed bone in any location.

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In the event of inconsistent usages between this document and any documents so incorporated by reference, the usage in this document controls. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim.

The above description is intended to be illustrative, and not restrictive. For example, the above-described examples (or one or more aspects thereof) may be used in combination with each other. Other embodiments can be used, such as by one of ordinary skill in the art upon reviewing the above description. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description as examples or embodiments, with each claim standing on its own as a separate embodiment, and it is contemplated that such embodiments can be combined with each other in various combinations or permutations. The scope of the invention is defined by the appended claims.

## Claims

1. A hard tissue implant assembly (100, 300, 400, 500) comprising:
an outer implant (104) securable to a cranium (52) of a patient; and
an inner implant (102) configured to engage with the outer implant (104) within the outer implant (104), the inner implant (102) being configured to be secured to the outer implant (104), **characterised in that** an inner edge (110) of the outer implant (104) is beveled and an outer edge (106) of the inner implant (102) is beveled complimentary to the beveled inner edge (110) of the outer implant (104) to configure the inner implant (102) for nesting within the outer implant (104).

2. The implant assembly of claim 1, further comprising:
a plurality of fasteners (112) securable to the inner implant (102) and the outer implant (104) to secure the inner implant (102) to the outer implant(104) and to secure the outer implant(104) to the cranium.

3. The implant assembly of claim 2, wherein the outer implant (104) is trimmable to reduce an overall shape and size of the outer implant (104) to form a trimmed portion (314), the trimmed portion (314) of the outer implant (104) securable to the cranium, and the inner implant (102) securable to the trimmed portion (314) of the outer implant (104) and to the cranium (52).

4. The implant assembly of any of claims 1-3, wherein the inner implant (102) and the outer implant (104) are manufactured through 3D printing.

5. The implant assembly of any of claims 1-4, wherein the inner implant (102) and the outer implant (104) are manufactured through selective laser sintering.

6. The implant assembly of any of claims 1-5, wherein the outer implant (504) is U-shaped.

7. The implant assembly of claim 6, wherein the outer implant (504) has an opening (522) on one side.

8. The implant assembly of any of claims 1-7, wherein the inner implant (102) is securable to a portion of the outer implant (104) and to the cranium.

## Patentansprüche

1. Hartgewebeimplantatanordnung (100, 300, 400, 500) umfassend:
ein äußeres Implantat (104), das an einem Schädel (52) eines Patienten sicherbar ist; und
ein inneres Implantat (102), das konfiguriert ist zum Ineingriffnehmen des äußeren Implantats (104) innerhalb des äußeren Implantats (104), wobei das innere Implantat (102) dazu konfiguriert ist, an dem äußeren Implantat (104) gesichert zu sein, **dadurch gekennzeichnet, dass** eine innere Kante (110) des äußeren Implantats (104) abgeschrägt ist und eine äußere Kante (106) des inneren Implantats (102) komplementär zu der abgeschrägten inneren Kante (110) des äußeren Implantats (104) abgeschrägt ist, um das innere Implantat (102) für das Verschachteln innerhalb des äußeren Implantats (104) zu konfigurieren.

2. Implantatanordnung nach Anspruch 1, ferner umfassend:
eine Vielzahl von Befestigungselementen (112), die an dem inneren Implantat (102) und dem äußeren Implantat (104) sicherbar sind, um das innere Implantat (102) an dem äußeren Implantat (104) zu sichern und um das äußere Implantat (104) an dem Schädel zu sichern.

3. Implantatanordnung nach Anspruch 2, wobei das äußere Implantat (104) zuschneidbar ist, um eine Gesamtform und -größe des äußeren Implantats (104) zu reduzieren, um einen zugeschnittenen Abschnitt (314) zu bilden, wobei der zugeschnittene Abschnitt (314) des äußeren Implantats (104) an dem Schädel sicherbar ist und das innere Implantat (102) an dem zugeschnittenen Abschnitt (314) des äußeren Implantats (104) und an dem Schädel (52) sicherbar ist.

4. Implantatanordnung nach einem der Ansprüche 1-3, wobei das innere Implantat (102) und das äußere Implantat (104) durch 3D-Drucken hergestellt werden.

5. Implantatanordnung nach einem der Ansprüche 1-4, wobei das innere Implantat (102) und das äußere Implantat (104) durch selektives Lasersintern hergestellt werden.

6. Implantatanordnung nach einem der Ansprüche 1-5, wobei das äußere Implantat (504) U-förmig ist.

7. Implantatanordnung nach Anspruch 6, wobei das äußere Implantat (504) eine Öffnung (522) auf einer Seite aufweist.

8. Implantatanordnung nach einem der Ansprüche 1-7, wobei das innere Implantat (102) an einem Abschnitt des äußeren Implantats (104) und an dem Schädel sicherbar ist.

## Revendications

1. Ensemble d'implants de tissus durs (100, 300, 400, 500) comprenant :
un implant externe (104) pouvant être fixé au crâne (52) d'un patient ; et
un implant interne (102) conçu pour venir en prise avec l'implant externe (104) à l'intérieur de l'implant externe (104), l'implant interne (102) étant conçu pour être fixé à l'implant externe (104), **caractérisé en ce qu'**un bord interne (110) de l'implant externe (104) est biseauté et un bord externe (106) de l'implant interne (102) est biseauté de manière complémentaire au bord interne biseauté (110) de l'implant externe (104) pour configurer l'implant interne (102) de manière à ce qu'il s'imbrique à l'intérieur de l'implant externe (104).

2. Ensemble d'implants selon la revendication 1, comprenant en outre :
une pluralité d'attaches (112) pouvant être fixées à l'implant interne (102) et à l'implant externe (104) destinées à fixer l'implant interne (102) à l'implant externe (104) et à fixer l'implant externe (104) au crâne.

3. Ensemble d'implants selon la revendication 2, ledit implant externe (104) pouvant être ajusté de manière à réduire la forme et la taille globales de l'implant externe (104) afin de former une partie ajustée (314), ladite partie ajustée (314) de l'implant externe (104) pouvant être fixée au crâne, et ledit implant interne (102) pouvant être fixé à la partie ajustée (314) de l'implant externe (104) et au crâne (52).

4. Ensemble d'implants selon l'une quelconque des revendications 1 à 3, ledit implant interne (102) et ledit implant externe (104) étant fabriqués par impression 3D.

5. Ensemble d'implants selon l'une quelconque des revendications 1 à 4, ledit implant interne (102) et ledit implant externe (104) étant fabriqués par frittage laser sélectif.

6. Ensemble d'implants selon l'une quelconque des revendications 1 à 5, ledit implant externe (504) étant en forme de U.

7. Ensemble d'implants selon la revendication 6, ledit implant externe (504) comportant une ouverture (522) sur un côté.

8. Ensemble d'implants selon l'une quelconque des revendications 1 à 7, ledit implant interne (102) pouvant être fixé à une partie de l'implant externe (104) et au crâne.
